(19) Europäisches Patentamt
European Patent Office
Office européen des brevets

(11) Veröffentlichungsnummer : **0 024 525**
**B1**

(12) # EUROPÄISCHE PATENTSCHRIFT

(45) Veröffentlichungstag der Patentschrift : **22.09.82**

(51) Int. Cl.$^3$ : **C 07 D277/68**

(21) Anmeldenummer : **80104081.7**

(22) Anmeldetag : **14.07.80**

(54) **Verfahren zur Herstellung von Luciferin und seinen Derivaten.**

(30) Priorität : **18.07.79 DE 2929115**

(43) Veröffentlichungstag der Anmeldung : **11.03.81 (Patentblatt 81/10)**

(45) Bekanntmachung des Hinweises auf die Patenterteilung : **22.09.82 Patentblatt 82/38**

(84) Benannte Vertragsstaaten : **AT BE CH DE FR GB IT LI LU NL SE**

(56) Entgegenhaltungen :
**JOURNAL OF THE CHEMICAL SOCIETY, PERKIN I, 1976, London N. SUZUKI et al. « Photochemical Decarboxylation of 2-Phenyl-Delta-2-thiazoline-4-carboxylic Acid »**

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Batz, Hans-Georg, Dr.**
**Traubinger Strasse 63**
**D-8132 Tutzing (DE)**
Erfinder : **Wulff, Karl, Dr.**
**Pütrichstrasse 18**
**D-8120 Weilheim (DE)**

(74) Vertreter : **Weickmann, Heinrich, Dipl.-Ing Patentanwälte Dipl.Ing.H.Weickmann et al**
**Dipl.Phys.Dr.K.Fincke Dipl.Ing.F.A.Weickmann**
**Dipl.Chem.B.Huber, Dr.-Ing.H.Liska Möhlstrasse 22**
**D-8000 München 86 (DE)**

**EP 0 024 525 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von Luciferin und seinen Derivaten

D-Luciferin entspricht der nachstehenden Formel II

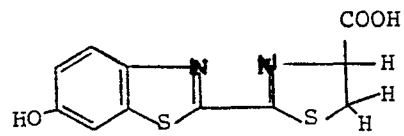

(II)

und stellt das Substrat des Enzyms Luciferase dar. Luciferin hat in letzter Zeit zunehmendes Interesse gefunden, da es als hochempfindliches Nachweisreagens in der klinisch-chemischen Analytik Anwendung findet.

Da die Isolierung des Luciferins, das heißt der D-(−)-2-(6′-hydroxy-2′-benzothiazolyl)-$\Delta^2$-thiazolin-4-carbonsäure, des für die Leuchtreaktion des Leuchtkäferchens verantwortlichen Substrats, aus natürlichen Quellen zu kostspielig ist, wurde schon bald nach der Strukturaufklärung mit Versuchen begonnen, diese Substanz zu synthetisieren [E.H. White, F. McCapra, G.F. Field and W.D. McElroy, J. Am. Chem. Soc. 83 (1961), 2402].

Die letzten beiden Stufen der Synthese von White et al. (loc. cit.), das heißt die Ätherspaltung, die von 2-Cyano-6-methoxybenzothiazol zu 2-Cyano-6-hydroxybenzothiazol führt, und die Umsetzung dieser letzteren Verbindung mit D-Cystein finden sich in allen bisher beschriebenen Synthesen wieder [siehe auch L.J. Bowie, Methods in Enzymology, Vol. LVII (1978) 15].

Variiert wurde bisher jeweils nur der Syntheseweg zur Schlüsselverbindung der Synthese, nämlich 2-Cyano-6-methoxybenzothiazol. Das einfachste Verfahren hierfür wurde 1965 publiziert [E.H. White, H. Wörther, G.F. Field and W.D. McElroy, J. Org. Chem. 30 (1965) 2344]. Dieses Verfahren führt, ausgehend von käuflichem 2-Amino-6-methoxybenzothiazol, über eine Diazotierung und eine Sandmeyer-Reaktion zu 2-Chlor-6-methoxybenzothiazol. Die kritischste Stufe der gesamten Synthese, nämlich die Ätherspaltung, erfolgt nach dieser Verfahrensweise und auch nach dem übrigen Stand der Technik mit Pyridinhydrochlorid. Mit diesem, wohl schonendsten, Reagens ließen sich Ausbeuten der Ätherspaltung von lediglich 15 bis 30 % erzielen.

Kürzlich berichteten T.L. Ho und G.A. Olah [Angewandte Chemie, internationale Ausgabe, 15 (1976) 774] über eine neue Methode der Ätherspaltung, bei der als Spaltungsreagens Jodtrimethylsilan oder eine Mischung aus Phenyltrimethylsilan und Jod verwendet wird. Diese Ätherspaltung ist jedoch nur für einfache Äther beschrieben, bei denen keine weiteren reaktionsfähigen Gruppen im Molekül vorliegen.

Da Luciferin nur sehr schwer zu reinigen ist, wie bereits von White et al. [J. Am. Chem. Soc. 83 (1961) 2402] festgestellt wurde, bestand die Aufgabe der Erfindung darin, ein Verfahren zur Herstellung von Luciferin anzugeben, mit dem es gelingt, diese Verbindung mit hohen Ausbeuten und mit einer sehr hohen Reinheit zu bilden, so daß aufwendige Reinigungsverfahren, wie die bislang angewandte Chromatographie, nicht mehr erforderlich sind.

Es hat sich nun überraschenderweise gezeigt, daß die von T.L. Ho und G.A. Olah beschriebene Ätherspaltungsmethode sich auch auf das nicht sehr beständige, multifunktionelle 2-Chlor-6-methoxybenzothiazol anwenden läßt und dies mit wesentlich besseren Ausbeuten unter Erzielung wesentlich reinerer Produkte, als es mit den herkömmlichen Verfahren möglich war. Dies war nicht zu erwarten, da der Fachmann in Anbetracht der bekannten Analogie zwischen Sauerstoffäther und Thioäthern eine Spaltung des Thiazolrings befürchten mußte. Diese Spaltung erfolgt überraschenderweise nicht, wenngleich die Reaktion am Stickstoffatom eintritt. Das Verfahren eignet sich in gleicher Weise für die analogen Verbindungen mit einer oder zwei Methoxygruppen im Benzolring.

Gegenstand der Erfindung ist daher ein Verfahren zur Herstellung von Luciferin und seinen Derivaten der allgemeinen Formel I

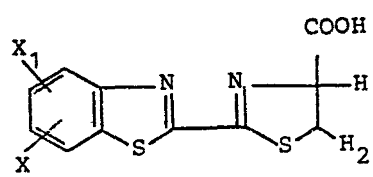

(I)

worin

X eine OH-Gruppe und

$X_1$ ein Wasserstoffatom oder eine OH-Gruppe bedeuten, durch Umsetzen von D-Cystein mit ausgehend von 2-Chlor-mono- bzw. -di-methoxybenzothiazol über eine Demethylierung gebildetem 2-Cyano-mono- oder -dihydroxybenzothiazol, welches dadurch gekennzeichnet ist, daß man die Demethylierung unter Feuchtigkeitsausschluß bei einer Temperatur von Raumtemperatur bis etwa 150 °C mit mindestens 2 Mol Jodtrimethylsilan oder einer Mischung aus Phenyltrimethylsilan und Jod durchführt

und das gebildete Silylderivat durch Behandeln mit einem aliphatischen Alkohol mit 1 bis 4 Kohlenstoffatomen und mit einer wässrigen Lösung eines Komplexierungsmittels für das bei der Reaktion freigesetzte Jod hydrolisiert.

Vorzugsweise führt man die Umsetzung mit Jodtrimethylsilan oder der Mischung aus Phenyltrimethylsilan und Jod unter Feuchtigkeitsausschluß bei einer Temperatur von etwa 70 bis etwa 110 °C durch. Mit Vorteil kann man diese Umsetzung in einem bei den Reaktionsbedingungen inerten organischen Lösungsmittel bei einer Temperatur von Raumtemperatur bis zur Rückflußtemperatur des Lösungsmittels durchführen. Als Lösungsmittel kann man insbesondere Chloroform, Methylenchlorid, Tetrachlorkohlenstoff und/oder Tetramethylensulfon verwenden.

Bei Einsatz einer Phenyltrimethylsilan/Jod-Mischung verwendet man vorzugsweise eine Mischung, die pro Mol Phenyltrimethylsilan 2 Grammatome Jod enthält.

Die Hydrolyse des durch die Umsetzung mit Jodtrimethylsilan oder der Mischung aus Phenyltrimethylsilan und Jod gebildeten Silylderivats erfolgt, indem man dieses zunächst mit einem aliphatischen Alkohol mit 1 bis 4 Kohlenstoffatomen, vorzugsweise mit Methanol, und dann mit einer wäßrigen Lösung eines Komplexierungsmittels für das bei der Reaktion freigesetzte Jod behandelt. Als Komplexierungsmittel verwendet man vorzugsweise Natriumthiosulfat, wobei man insbesondere eine 1- bis 20 gew.-%-ige, vorzugsweise eine 5- bis 10 gew.-%-ige wäßrige Natriumthiosulfatlösung einsetzt.

Unter Anwendung dieser milden Hydrolyse, die vorzugsweise bei Raumtemperatur durchgeführt wird, kann die Nebenreaktion mit Methanol nahezu vollständig unterdrückt werden, wodurch es gelingt, die gewünschten Endprodukte mit hoher Reinheit zu gewinnen.

Mit Vorteil kann man das erfindungsgemäße Verfahren unter verschiedenartiger Abfolge der Umsetzungsschritte, nämlich der Bildung des Silylderivats, der Hydrolyse des Silylderivats und der Einführung der Cyanogruppe in die 2-Stellung, durchführen. Diese verschiedenen Reaktionsmöglichkeiten ergeben sich aus dem nachstehenden Reaktionsschema am Beispiel des Luciferins :

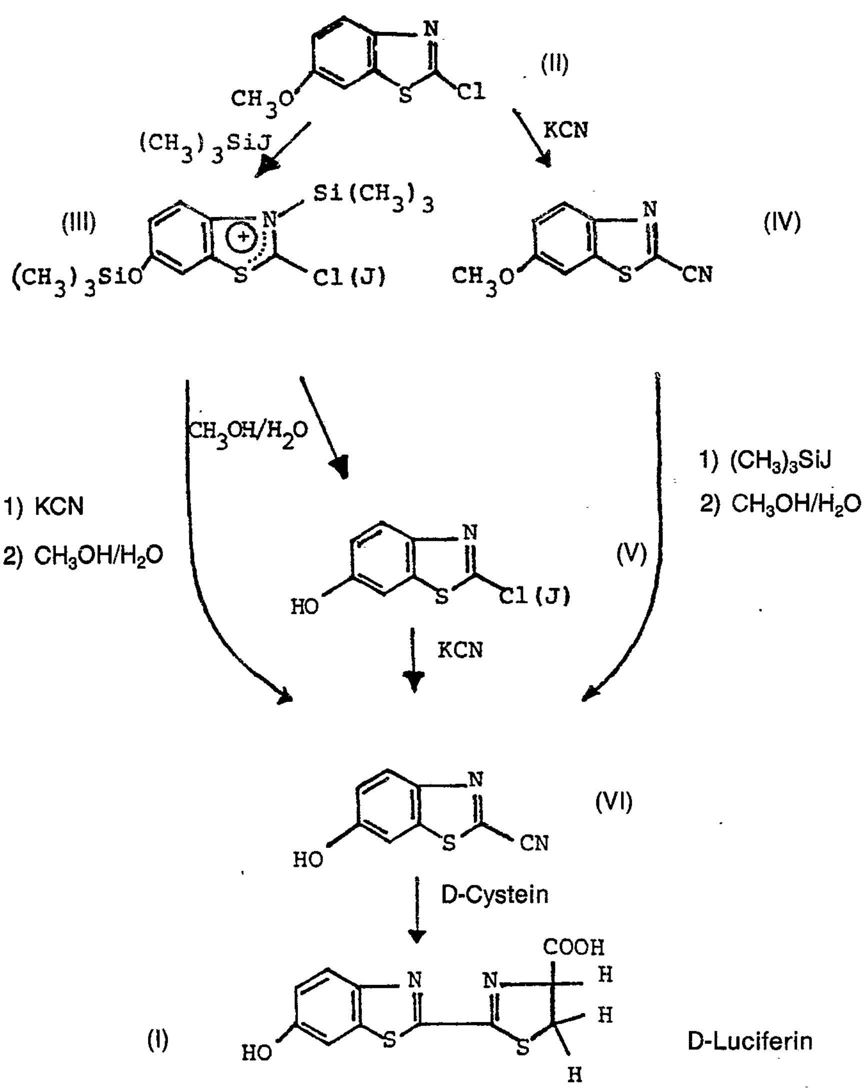

Gemäß einer ersten bevorzugten Ausführungsform des erfindungsgemäßen Verfahrens setzt man zunächst 2-Chlor-mono- oder -dimethoxybenzothiazol (II) mit

a) Jodtrimethylsilan um,

b) hydrolysiert dann das erhaltene Produkt, was man vorzugsweise durch Behandeln mit Methanol und einer wäßrigen Natriumthiosulfatlösung bewirkt, worauf man

c) das gebildete Gemisch aus 2-Chlor-mono- oder -dihydroxybenzothiazol und 2-Jod-mono- oder -dihydroxybenzothiazol (V) mit einem Alkalimetallcyanid, vorzugsweise Kaliumcyanid, umsetzt.

Gemäß einer weiteren bevorzugten Ausführungsform des erfindungsgemäßen Verfahrens setzt man 2-Chlor-mono- oder -dimethoxybenzothiazol zunächst

a) mit Jodtrimethylsilan um und

b) bringt das erhaltene Produkt mit einem Alkalimetallcyanid, vorzugsweise mit Kaliumcyanid, zur Reaktion, bevor man

c) das erhaltene Produkt hydrolysiert, was man vorzugsweise mit Methanol und einer wäßrigen Natriumthiosulfatlösung bewirkt.

Schließlich kann man das erfindungsgemäße Verfahren in der Weise durchführen, daß man 2-Chlor-mono- oder -dimethoxybenzothiazol

a) mit einem Alkalimetallcyanid, vorzugsweise Kaliumcyanid, umsetzt,

b) das gebildete 2-Cyano-mono- oder -dimethoxybenzothiazol (IV) mit Jodtrimethylsilan umsetzt und

c) das erhaltene Produkt hydrolysiert, was man vorzugsweise mit Methanol und einer wäßrigen Natriumthiosulfatlösung erreicht.

Bei diesen bevorzugten Ausführungsformen des erfindungsgemäßen Verfahrens bewirkt man die Umsetzung mit dem Alkalimetallcyanid vorzugsweise in einem inerten organischen Lösungsmittel, insbesondere in Dimethylsulfoxid, und in Gegenwart eines Phasen-Transfer-Katalysators. Dabei kann man als Phasen-Transfer-Katalysator ein quartäres Alkylammoniumsalz, vorzugsweise ein Tetraalkylammoniumsalz, wobei die Alkylgruppen 1 bis 4 Kohlenstoffatomen aufweisen, wie Tetrabutylammoniumbromid, oder einen Kronenäther, wie insbesondere 18-Crown-6, verwenden. Dabei hat sich die Katalyse mit Hilfe des Phasen-Transfer-Katalysators insbesondere für die Methoxy-Verbindung als besonders wirksam erwiesen. Diese Katalyse ist auch bei der Umsetzung des als Zwischenprodukt gebildeten N-Trimethylsilyl-mono- oder -ditrimethylsilyloxy-benzothiazols von Vorteil, da es in dieser Weise gelingt, beide Stufen zusammenzufassen.

Überraschenderweise hat sich gezeigt, daß bei der erfindungsgemäßen Verfahrensweise das gebildete Hydroxy-2-cyanobenzothiazol bei der angewandten einfachen Reaktionsfolge reiner anfällt als bei jeder anderen vorbekannten Synthese. Somit sind aufwendige Reinigungsverfahren, wie die chromatographische Reinigung, nicht mehr notwendig, und die anschließende Kupplung mit D-Cystein führt zu hochreinem D-Luciferin bzw. Analogen davon.

Als besonders vorteilhaft hat es sich weiterhin erwiesen, das Produkt dadurch zu reinigen, daß man es mit in mit Stickstoff begastem Wasser suspendiert, die Suspension mit Natriumbicarbonat versetzt, die erhaltene Lösung mit einem inerten organischen Lösungsmittel, wie insbesondere Essigester, extrahiert, die wäßrige Phase ansäuert, wozu man beispielsweise Chlorwasserstoffsäure verwendet, und zur Kristallisation stehen läßt. In dieser Weise erhält man chromatographisch reines Produkt.

Die Erfindung sei im folgenden näher anhand der nachstehenden Beispiele erläutert.

## Beispiel 1

2-Chlor-6-methoxybenzothiazol

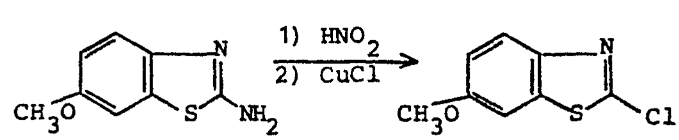


Man löst 54 g (0,3 Mol) 2-Amino-6-methoxybenzothiazol in 150 ml Ameisensäure, 60 ml Eisessig und 120 ml konzentrierter Chlorwasserstoffsäure. Dann gibt man unter Rühren bei −5 °C tropfenweise eine Lösung von 21 g Natriumnitrit und 30 ml Wasser zu. Man rührt die rotbraune Lösung während 15 Minuten bei 0 °C. Anschließend trägt man die Lösung portionsweise bei 0 °C in eine heftig gerührte Lösung von 39 g (0,39 Mol) Kupfer (I)-chlorid in 180 ml 28 %-iger Chlorwasserstoffsäure ein. Man beobachtet eine starke Gasentwicklung. Anschließend läßt man die Temperatur der Mischung auf Raumtemperatur ansteigen und rührt über Nacht bei dieser Temperatur. Dann gießt man die Lösung in etwa 1 l Eiswasser, saugt das ausgefallene Produkt ab und wäscht es mit Wasser.

Das feuchte Produkt wird in etwa 800 ml Methanol aufgenommen, gerührt und von unlöslichen Anteilen abgetrennt. Man versetzt das Filtrat mit Kohle, filtriert und zieht das organische Lösungsmittel im Vakuum bei 50 °C ab. Die zurückbleibende Suspension wird mit Wasser verdünnt und abgesaugt. Man löst das feuchte Produkt in etwa 600 ml Essigester (wobei wenig ungelöstes Material zurückbleibt), trocknet mit Natriumsulfat, gibt Kohle zu und saugt ab. Man dampft das Filtrat im Vakuum zur Trockne ein und trocknet den Rückstand im Exsikkator.

Man erhält 32,75 g 2-Chlor-6-methoxybenzothiazol, was einer Ausbeute von 54,6 % der Theorie entspricht (nach der Literatur erhält man eine Ausbeute von lediglich 25 bis 45 %).

Die Verbindung besitzt einen Schmelzpunkt von 48 bis 49 °C und ist chromatographisch rein.

Beispiel 2

2-Chlor-6-hydroxybenzothiazol

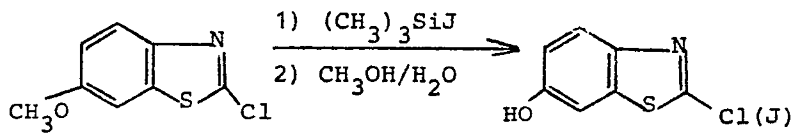


Man löst 10 g (0,05 Mol) des gemäß Beispiel 1 hergestellten 2-Chlor-6-methoxybenzothiazols in 30 ml absolutem Chloroform. Dann gibt man 21 g (etwa 0,1 Mol, 15 ml) Jodtrimethylsilan zu und rührt bei einer Badtemperatur von 80 °C unter Feuchtigkeitsausschluß unter Rückfluß. Nach einer Reaktionszeit von 70 Stunden gibt man weitere 4 ml Jodtrimethylsilan zu und rührt während weiterer 40 Stunden bei der Rückflußtemperatur.

Anschließend suspendiert man die Reaktionslösung in etwa 400 ml Chloroform, gibt 100 ml Methanol zu, schüttelt kurz und erhält eine klare Lösung. Man gibt sofort 300 ml einer 10 %-igen wäßrigen Natriumthiosulfatlösung zu und schüttelt. Man trennt die Chloroformphase ab und extrahiert die wäßrige Phase zweimal mit Chloroform. Man trocknet die vereinigten Extrakte mit Natriumsulfat, versetzt mit Kohle, filtriert und dampft im Vakuum ein.

Man erhält 8,4 g eines Rohprodukts, das man in 300 ml Wasser suspendiert, mit 20 ml 2 n Natriumhydroxidlösung versetzt und schüttelt. Nach kurzer Zeit wird eine geringe Menge eines unlöslichen Produkts abgesaugt und mit Wasser gewaschen. Das trübe Filtrat wird mit Kohle versetzt und abgesaugt, worauf man das Filtrat mit 2 n Chlorwasserstoffsäure ansäuert. Das ausgefallene Produkt wird dann abgesaugt, mit Wasser gewaschen und im Exsikkator getrocknet.

Man erhält 6,28 g eines Gemisches aus 2-Chlor-6-hydroxybenzothiazol und 2-Jod-6-hydroxybenzothiazol, was einer Ausbeute von 67,6 % der Theorie entspricht (nach den in der Literatur beschriebenen Verfahrensweisen erhält man diese Verbindung mit einer Ausbeute von lediglich 15 bis 32 %). Das Material schmilzt bei 160 bis 164 °C, ist chromatographisch rein und entspricht den Literaturangaben.

Beispiel 3

2-Cyano-6-hydroxybenzothiazol

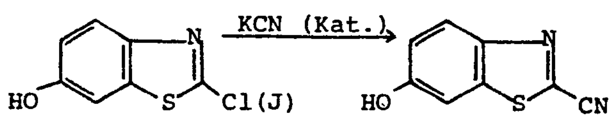


Man gibt 4 g des gemäß Beispiel 2 erhaltenen Gemisches aus 2-Chlor-6-hydroxybenzothiazol und 2-Jod-6-hydroxybenzothiazol zu einer auf 120 °C erhitzten Suspension von 3 g Kaliumcyanid und 100 mg 18-Crown-6 in 150 ml Dimethylsulfoxid und rührt bei einer Badtemperatur von 120 °C. Nach einer Reaktionszeit von 3 Stunden gießt man die Reaktionsmischung in 1 l Eiswasser und säuert die erhaltene Lösung mit verdünnter Chlorwasserstoffsäure an. Anschließend extrahiert man zweimal mit jeweils 500 ml Äther, wäscht die Extrakte mit Wasser, trocknet über Natriumsulfat, versetzt mit Kohle, filtriert und engt das Filtrat im Vakuum ein. Man erhält 2,5 g rohes 2-Cyano-6-hydroxybenzothiazol.

Man suspendiert das Rohprodukt in 100 ml Wasser, versetzt mit einer 1 n Natriumhydroxidlösung, wobei sich das Material in kurzer Zeit löst und wenig ungelöstes zurückbleibt. Man gibt Kohle zu, saugt ab, säuert das Filtrat mit verdünnter Chlorwasserstoffsäure an, saugt das ausgefallene Kristallisat ab und trocknet es im Exsikkator. Man löst das Produkt in 150 ml Äther, wäscht mit 100 ml Ligroin (zur Ausfällung von Verunreinigungen), versetzt mit Kohle und saugt ab. Man dampft das Filtrat im Vakuum ein und erhält 2,0 g 2-Cyano-6-hydroxybenzothiazol, was einer Ausbeute von 52 % der Theorie entspricht. Die Verbindung schmilzt bei 190 bis 200 °C unter Zersetzung.

Beispiel 4

D-Luciferin

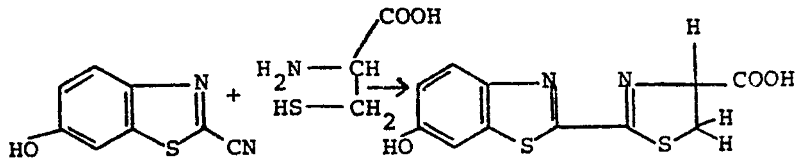

Man löst 685 mg (3,9 mMol) D-Cystein-hydrochlorid-monohydrat in 13 ml Wasser, das man zuvor mit Stickstoff begast hat. Dann stellt man unter Stickstoff mit etwa 6 ml einer 1 n Natriumhydroxidlösung auf einen pH-Wert von 8 und gibt dann eine Lösung von 528 mg (3 mMol) 2-Cyano-6-hydroxybenzothiazol in 22 ml Methanol (analysenrein) unter Stickstoff zu. Man rührt die gelbe Lösung unter einer Stickstoffatmosphäre und unter Lichtausschluß während 1 Stunde bei Raumtemperatur. Anschließend säuert man mit 3 ml einer 2 n Chlorwasserstoffsäure an und läßt etwa 3 Stunden bei 4 °C stehen. Das ausgefallene Kristallisat wird unter einer Stickstoffatmosphäre abgesaugt, mit Methanol und Wasser (1 : 1) gewaschen und im Exsikkator unter Lichtausschluß getrocknet.

Man erhält 0,75 g D-Luciferin, was einer Ausbeute von 89,3 % der Theorie entspricht. Die Verbindung schmilzt bei 187 bis 188 °C unter Zersetzung und ist chromatographisch identisch mit dem Literaturprodukt, enthält jedoch geringe Verunreinigungen.

Man suspendiert 150 mg dieses Rohprodukts in 8 ml mit Stickstoff begastem Wasser, versetzt mit Natriumbicarbonat und erhält nach kurzer Zeit eine Lösung. Die Lösung extrahiert man sofort mit Essigester, trennt die organische Phase ab und säuert die wäßrige Phase mit einer 2 n Chlorwasserstoffsäure an. Nach einstündigem Stehen bei 4 °C saugt man das Kristallisat ab und trocknet es im Exsikkator. Man erhält 0,1 g chromatographisch reines D-Luciferin, das sich als Luciferasesubstrat identisch verhält wie das Literaturprodukt.

## Beispiel 5

2-Cyano-6-hydroxybenzothiazol

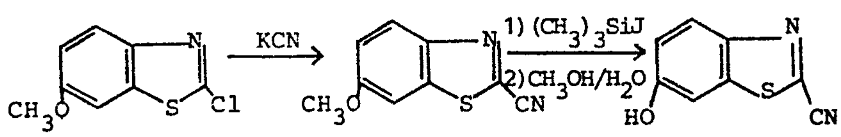


Man rührt 4 g (0,02 Mol) 2-Chlor-6-methoxybenzothiazol und 2,6 g (0,04 Mol) Kaliumcyanid in 150 ml Dimethylsulfoxid während 3 Stunden bei 100 °C. Dann gießt man die abgekühlte Lösung in etwa 1 l Wasser, extrahiert zweimal mit Äther, wäscht den Ätherextrakt mit Wasser, trocknet ihn mit Natriumsulfat und dampft ihn ein.

Den Eindampfrückstand löst man in 5 ml Chloroform, versetzt mit 6 ml Jodtrimethylsilan und rührt während etwa 100 Stunden bei einer Badtemperatur von 85 °C. Dann verdünnt man die Lösung mit Chloroform, gibt etwas Methanol zu und schüttelt kurz. Anschließend schüttelt man mit einer 5 %-igen Natriumthiosulfatlösung, trennt die organische Phase ab und extrahiert die wäßrige Phase mit Chloroform. Die vereinigten Chloroformextrakte werden mit Natriumsulfat getrocknet und eingedampft. Man reinigt das Produkt über einer mit Kieselgel beschickten Säule unter Verwendung einer Tetrahydrofuran/Isopropyläther-Mischung (1/4) als Elutionsmittel.

Man erhält 1,5 g 2-Cyano-6-hydroxybenzothiazol, was einer Ausbeute von 45 % der Theorie entspricht. Die Verbindung schmilzt bei 190 bis 200 °C unter Zersetzung.

Aus den obigen Beispielen ist ohne weiteres zu erkennen, daß das erfindungsgemäße Verfahren mit wesentlich besseren Ausbeuten zu einem wesentlich reineren Produkt, nämlich D-Luciferin, führt und damit eine erhebliche Bereicherung der Technik darstellt.

## Beispiel 6

2-Cyano-6-hydroxybenzothiazol (Variante 3)

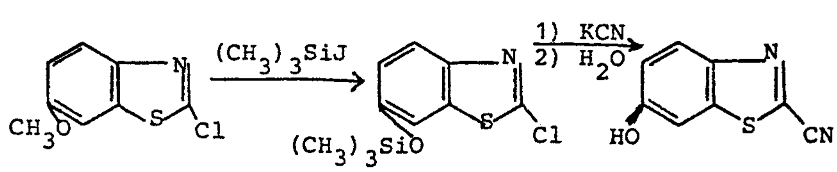


3 g (0,015 Mol) 2-Chlor-6-methoxybenzothiazol werden in 9 ml absolutem Chloroform gelöst und mit 6 g = 4,5 ml (0,03 Mol) Jodtrimethylsilan versetzt. Die Lösung wird bei 80 °C Badtemperatur unter Rückfluß gerührt.

Nach 25 Stunden werden nochmals 3 g = 2,1 ml Jodtrimethylsilan zugegeben und unter Rückfluß gerührt. Nach weiteren 30 Stunden Reaktionszeit wird die Lösung im Vakuum eingedampft, der Rückstand in 100 ml DMSO gelöst und mit 3 g KCN (3-fache Mol-Menge) versetzt. Die Suspension wird dann bei 100 °C gerührt.

Nach 3 Stunden Reaktionszeit wird in ca. 800 ml Eiswasser gegossen, mit 2 n HCl angesäuert, einmal mit 500 ml und einmal mit 300 ml Äther extrahiert. Der Extrakt wird mit Wasser gewaschen, mit Natriumsulfat getrocknet und eingedampft.

Die noch schwach verunreinigte Substanz wird über Kieselgel 60 mit Isopropyläther und THF 4 : 1 gereinigt. Ausbeute : 42 %.

Beispiel 7

Das Verfahren der Beispiele 1 bis 4 wird wiederholt, jedoch wird anstelle von 2-Amino-6-methoxybenzothiazol eine äquivalente Menge 2-Amino-4,6-dimethoxybenzothiazol eingesetzt. Die Jodtrimethylsilan-Menge beträgt 0,15 Mol. Man erhält so 4'-Hydroxyluciferin.

**Ansprüche**

1. Verfahren zur Herstellung von Luciferin und seinen Derivaten der allgemeinen Formel I

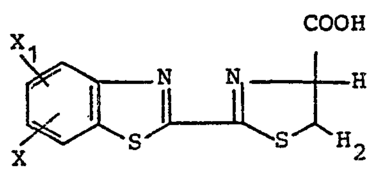

(I)

worin

X eine OH-Gruppe und

$X_1$ ein Wasserstoffatom oder eine OH-Gruppe bedeuten, durch Umsetzen von D-Cystein mit ausgehend von 2-Chlor-mono- bzw. -di-methoxy-benzothiazol über eine Demethylierung gebildetem 2-Cyano-mono- oder -dihydroxybenzothiazol, dadurch gekennzeichnet, daß man die Demethylierung unter Feuchtigkeitsausschluß bei einer Temperatur von Raumtemperatur bis etwa 150 °C mit mindestens 2 Mol Jodtrimethylsilan oder einer Mischung aus Phenyltrimethylsilan und Jod durchführt und das gebildete Silylderivat durch Behandeln mit einem aliphatischen Alkohol mit 1 bis 4 Kohlenstoffatomen und mit einer wässrigen Lösung eines Komplexierungsmittels für das bei der Reaktion freigesetzte Jod hydrolisiert.

2. Verfahren nach Anspruch 1, dadurch gekennzeichnet, daß man die Umsetzung in einem inerten organischen Lösungsmittel bei einer Temperatur von Raumtemperatur bis zur Rückflußtemperatur des Lösungsmittels durchführt.

3. Verfahren nach Anspruch 2, dadurch gekennzeichnet, daß man als Lösungsmittel Chloroform, Methylenchlorid, Tetrachlorkohlenstoff und/oder Tetramethylensulfon verwendet.

4. Verfahren nach Anspruch 1, dadurch gekennzeichnet, daß man als aliphatischen Alkohol Methanol verwendet.

5. Verfahren nach Anspruch 1, dadurch gekennzeichnet, daß man als Komplexierungsmittel Natriumthiosulfat verwendet.

6. Verfahren nach den Ansprüchen 4 und 5, dadurch gekennzeichnet, daß man die Hydrolyse bei Raumtemperatur bewirkt.

7. Verfahren nach den Ansprüchen 1 bis 6, dadurch gekennzeichnet, daß man 2-Chlor-mono- oder -dimethoxybenzothiazol

a) mit Jodtrimethylsilan umsetzt,

b) das erhaltene Produkt hydrolysiert und

c) das gebildete Gemisch aus 2-Chlor-6-hydroxybenzothiazol und 2-Jod-6-hydroxybenzothiazol mit einem Alkalimetallcyanid, vorzugsweise mit Kaliumcyanid,

umsetzt.

8. Verfahren nach den Ansprüchen 1 bis 6, dadurch gekennzeichnet, daß man 2-Chlor-mono- oder -dimethoxybenzothiazol

a) mit Jodtrimethylsilan umsetzt,

b) das erhaltene Produkt mit einem Alkalimetallcyanid, vorzugsweise Kaliumcyanid, umsetzt und

c) das erhaltene Produkt hydrolysiert.

9. Verfahren nach den Ansprüchen 1 bis 6, dadurch gekennzeichnet, daß man 2-Chlor-mono- oder -dimethoxybenzothiazol

a) mit einem Alkalimetallcyanid, vorzugsweise Kaliumcyanid, umsetzt,

b) das gebildete 2-Cyano-mono- oder -dimethoxy-benzothiazol mit Jodtrimethylsilan umsezt und

c) das erhaltene Produkt hydrolysiert.

10. Verfahren nach den Ansprüchen 7 bis 9, dadurch gekennzeichnet, daß man die Umsetzung mit dem Alkalimetallcyanid in einem inerten organischen Lösungsmittel, vorzugsweise in Dimethylsulfoxid, und in Gegenwart eines Phasen-Transfer-Katalysators durchführt.

11. Verfahren nach Anspruch 10, dadurch gekennzeichnet, daß man als Phasen-Transfer-Katalysator ein quartäres Alkylammoniumsalz oder einen Kronenäther verwendet.

12. Verfahren nach Anspruch 11, dadurch gekennzeichnet, daß man als Kronenäther 18-Crown-6 verwendet.

13. Verfahren nach mindestens einem der vorhergehenden Ansprüche, dadurch gekennzeichnet, daß man das gebildete Luciferin bzw. Luciferinderivat in mit Stickstoff begastem Wasser suspendiert und mit Natriumbicarbonat versetzt, worauf man die erhaltene Lösung mit einem inerten organischen Lösungsmittel, vorzugsweise mit Essigester, extrahiert und die wäßrige Phase ansäuert und zur Kristallisation stehen läßt.

14. Verfahren nach einem der vorhergehenden Ansprüche, dadurch gekennzeichnet, daß man 2-Chlor-6-methoxybenzothiazol oder 2-Chlor-4,6-dimethoxybenzothiazol einsetzt.

**Claims**

1. Process for the preparation of luciferin and its derivatives of the general formula I

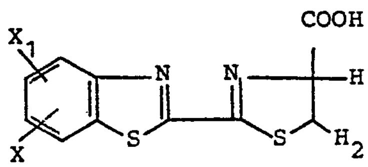

(I)

wherein

X signifies an OH group and

$X_1$ a hydrogen atom or an OH group, by the reaction of D-cysteine with 2-cyanomono- or -dihydroxybenzothiazole obtained from 2-chloromono- or -dimethoxybenzothiazole *via* a demethylation, characterised in that one carries out the demethylation, with the exclusion of moisture at a temperature of from room temperature to about 150 °C, with at least 2 mole of iodotrimethylsilane or of a mixture of phenyltrimethylsilane and iodine and hydrolyses the silyl derivative formed by treatment with an aliphatic alcohol with 1 to 4 carbon atoms and with an aqueous solution of a complexing agent for the iodine liberated by the reaction.

2. Process according to claim 1, characterised in that one carries out the reaction in an inert organic solvent at a temperature of from room temperature to the reflux temperature of the solvent.

3. Process according to claim 2, characterised in that, as solvent, one uses chloroform, methylene chloride, carbon tetrachloride and/or tetramethylenesulphone.

4. Process according to claim 1, characterised in that, as aliphatic alcohol, one uses methanol.

5. Process according to claim 1, characterised in that, as complexing agent, one uses sodium thiosulphate.

6. Process according to claims 4 and 5, characterised in that one carries out the hydrolysis at room temperature.

7. Process according to claims 1 to 6, characterised in that one reacts 2-chloromono- or -dimethoxybenzothiazole :

a) with iodotrimethylsilane,

b) hydrolyses the product obtained and

c) reacts the mixture of 2-chloro-6-hydroxybenzothiazole and 2-iodo-6-hydroxybenzothiazole formed with an alkali metal cyanide, preferably with potassium cyanide.

8. Process according to claims 1 to 6, characterised in that one reacts 2-chloromono- or -dimethoxybenzothiazole

a) with iodotrimethylsilane,

b) reacts the product obtained with an alkali metal cyanide, preferably potassium cyanide and

c) hydrolyses the product obtained.

9. Process according to claims 1 to 6, characterised in that one reacts 2-chloromono- or -dimethoxybenzothiazole

a) with an alkali metal cyanide, preferably potassium cyanide,

b) reacts the 2-cyanomono- or -dimethoxybenzothiazole formed with iodotrimethylsilane and

c) hydrolyses the product obtained.

10. Process according to claims 7 to 9, characterised in that one carries out the reaction with the alkali metal cyanide in an inert organic solvent, preferably in dimethyl sulphoxide, and in the presence of a phase-transfer catalyst.

11. Process according to claim 10, characterised in that, as phase transfer catalyst, one uses a

quaternary alkyl ammonium salt or a crown ether.

12. Process according to claim 11, characterised in that, as crown ether, one uses 18-crown-6.

13. Process according to at least one of the preceding claims, characterised in that one suspends the luciferin or luciferin derivative obtained in water gassed with nitrogen and mixes with sodium bicarbonate, whereupon one extracts the solution obtained with an inert organic solvent, preferably with ethyl acetate, and acidifies the aqueous phase and leaves to crystallise.

14. Process according to one of the preceding claims, characterised in that one uses 2-chloro-6-methoxybenzothiazole or 2-chloro-4,6-dimethoxybenzothiazole.

## Revendications

1. Procédé pour la préparation de la luciférine et de ses dérivés de formule générale I

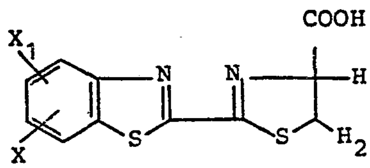

(I)

dans laquelle

X signifie un groupe OH et

$X_1$ un atome d'hydrogène ou un groupe OH, par réaction de la D-cystéine avec du 2-cyano-mono- ou dihydroxybenzothiazole formé à partir du 2-chloro-mono- ou respectivement diméthoxybenzothiazole par une déméthylation, caractérisé en ce que l'on effectue la déméthylation à l'abri de l'humidité à une température allant de la température ambiante à environ 150 °C avec au moins 2 moles d'iodotriméthylsilane ou d'un mélange de phényltriméthylsilane et d'iode et que l'on hydrolyse le dérivé sylilé formé par traitement par un alcool aliphatique avec 1 à 4 atomes de carbone et par une solution aqueuse d'un agent complexant pour l'iode libéré dans la réaction.

2. Procédé selon la revendication 1, caractérisé en ce que l'on effectue la réaction dans un solvant organique inerte à une température allant de la température ambiante à la température de reflux du solvant.

3. Procédé selon la revendication 2, caractérisé en ce que l'on utilise comme solvant le chloroforme, le chlorure de méthylène, le tétrachlorure de carbone et/ou la tétraméthylènesulfone.

4. Procédé selon la revendication 1, caractérisé en ce que l'on utilise comme alcool aliphatique le méthanol.

5. Procédé selon la revendication 1, caractérisé en ce que l'on utilise comme agent complexant le thiosulfate de sodium.

6. Procédé selon les revendications 4 et 5, caractérisé en ce que l'on effectue l'hydrolyse à température ambiante.

7. Procédé selon les revendications 1 à 6, caractérisé en ce que :

a) on fait réagir le 2-chloro-mono- ou diméthoxybenzothiazole avec l'iodotriméthylsilane,

b) on hydrolyse le produit obtenu et

c) on fait réagir le mélange formé de 2-chloro-6-hydroxybenzothiazole et de 2-iodo-6-hydroxybenzothiazole avec un cyanure alcalin, de préférence le cyanure de potassium.

8. Procédé selon les revendications 1 à 6, caractérisé en ce que :

a) on fait réagir le 2-chloro-mono ou diméthoxybenzothiazole avec l'iodotriméthylsilane,

b) on fait réagir le produit obtenu avec un cyanure alcalin, de préférence le cyanure de potassium, et

c) on hydrolyse le produit obtenu.

9. Procédé selon les revendications 1 à 6, caractérisé en ce que :

a) on fait réagir le 2-chloro-mono- ou diméthoxybenzothiazole avec un cyanure alcalin, de préférence le cyanure de potassium,

b) on fait réagir le 2-cyano-mono- ou diméthoxybenzothiazole formé avec l'iodotriméthylsilane et

c) on hydrolyse le produit obtenu.

10. Procédé selon les revendications 7 à 9, caractérisé en ce que l'on effectue la réaction avec le cyanure alcalin dans un solvant organique inerte, de préférence dans le diméthylsulfoxyde, et en présence d'un catalyseur de transfert de phases.

11. Procédé selon la revendication 10, caractérisé en ce que l'on utilise comme catalyseur de transfert de phases un sel d'alcoylammonium quaternaire ou un éther-couronne.

12. Procédé selon la revendication 11, caractérisé en ce que l'cn utilise comme éther-couronne le 18-couronne-6.

13. Procédé selon au moins une des revendications précédentes, caractérisé en ce que l'on met en suspension la luciférine ou respectivement le dérivé de luciférine formé dans de l'eau gazéifiée par l'azote

et que l'on ajoute du bicarbonate de sodium, après quoi on extrait la solution obtenue par un solvant organique inerte, de préférence de l'acétate d'éthyle, et on acidifie la phase aqueuse et on la laisse reposer pour la cristallisation.

14. Procédé selon l'une des revendications précédentes, caractérisé en ce que l'on utilise le 2-chloro-6-méthoxybenzothiazole ou le 2-chloro-4,6-diméthoxybenzothiazole.